(19)

**Europäisches Patentamt**
**European Patent Office**
**Office européen des brevets**

(11) **EP 1 633 373 B1**

(12) **EUROPEAN PATENT SPECIFICATION**

(45) Date of publication and mention
of the grant of the patent:
**21.07.2010 Bulletin 2010/29**

(51) Int Cl.:
**A61K 31/716** (2006.01)    **A23K 1/16** (2006.01)
**A61P 37/04** (2006.01)

(21) Application number: **04735831.2**

(22) Date of filing: **02.06.2004**

(86) International application number:
**PCT/NO2004/000157**

(87) International publication number:
**WO 2004/105775 (09.12.2004 Gazette 2004/50)**

(54) **PHARMACEUTICAL COMPOSITION COMPRISING GLUCAN DERIVED FROM MICROALGAE**

PHARMAZEUTISCHE ZUSAMMENSETZUNG ENTHALTEND GLUCAN AUS MIKROALGEN

COMPOSITION PHARMACEUTIQUE COMPRENANT GLUCANE DERIVE DE MICROALGUES

(84) Designated Contracting States:
**AT BE BG CH CY CZ DE DK EE ES FI FR GB GR
HU IE IT LI LU MC NL PL PT RO SE SI SK TR**

(30) Priority: **03.06.2003 NO 20032494**

(43) Date of publication of application:
**15.03.2006 Bulletin 2006/11**

(73) Proprietor: **Sinvent AS
7465 Trondheim (NO)**

(72) Inventors:
• **STÖRSETH, Trond, Rövik
N-7052 Trondheim (NO)**
• **SKJERMO, Jorunn
N-7045 Trondheim (NO)**

(74) Representative: **Hayes, Adrian Chetwynd
Boult Wade Tennant
Verulam Gardens
70 Gray's Inn Road
London WC1X 8BT (GB)**

(56) References cited:
**WO-A1-02/04000      DE-A1- 19 608 563
US-A- 5 084 386      US-A- 5 574 023
US-A- 5 866 150**

• **VISMARA R. ET AL.: 'Natural vitamin E
enrichment of Artemia salina fed freshwater and
marine microalgae' JOURNAL OF APPLIED
PHYCOLOGY vol. 15, 2003, pages 75 - 80,
XP009031225**

• **D'SOUZA F.M.L. ET AL.: 'Flocculated microalgae
concentrates as diets for larvae of the tiger prawn
Penaeus monodon Fabricius' AQUACULTURE
NUTRITION vol. 8, no. 2, 2002, pages 113 - 120,
XP002903872**

• **LEBEAU T. ET AL.: 'Alginate-entrapped Haslea
ostrearia as inoculum for the greening of oysters'
BIOTECHNOLOGY TECHNIQUES vol. 12, no. 11,
November 1998, pages 847 - 850, XP002903873**

• **GRANUM E.: 'Metabolism and function of Beta-
1,3-glucan in marine diatoms' January 2002,
DEPARTMENT OF BIOTECHNOLOGY, FACULTY
OF NATURAL SCIENCES AND TECHNOLOGY,
NORWEGIAN UNIVERSITY OF SCIENCE AND
TECHNOLOGY (NTNU), TRONDHEIM, pages I -
VII, 1 - 48, PAPER 1 - 5 (URN:NBN:NO-2102),
XP002903874**

• **RAA J. ET AL.: 'The use of immune-stimulants in
fish and shellfish feeds' AVANCES EN
NUTRICION ACUICOLA V. MEMORIAS DEL V
SIMPOSIUM INTERNACIONAL DE NUTRICION
ACUICOLA 19 November 2000 - 22 November
2000, pages 47 - 56, XP002903875**

• **PATENT ABSTRACTS OF JAPAN vol. 007, no. 086
(C-161) 09 April 1983 & JP 58 015 920 A
(KURORERA KOGYO KK) 29 January 1983**

• **DELAPORTE M. ET AL.: 'Effect of a mono-specific
algal diet on immune functions in two bivalve
species - Crassostrea gigas and Ruditapes
philippinarum' THE JOURNAL OF
EXPERIMENTAL BIOLOGY vol. 206, 2003, pages
3053 - 3064, XP002903876**

**(Cont. next page)**

- **STORSETH T.R. ET AL.: 'Characterization of a Beta-D-(1-3)-glucan from the marine diatom Chaetoceros mülleri by high-resolution magic-angle spinning NMR spectroscopy on whole algal cells' CARBOHYDRATE RESEARCH vol. 339, 2004, pages 421 - 424, XP004482702**

- **JAMOIS FRANK ET AL: "Glucan-like synthetic oligosaccharides: iterative synthesis of linear oligo-beta-(1,3)-glucans and immunostimulatory effects." GLYCOBIOLOGY APR 2005, vol. 15, no. 4, April 2005 (2005-04), pages 393-407, ISSN: 0959-6658**

**Description**

**Field of the invention**

The present invention describes a pharmaceutical composition, a compound, feed and constituent of feed having immunostimulating, prophylactic and/or growth enhancing effects for use in enhancing the overall resistance against diseases caused by pathogenic and opportunistic pathogenic microorganisms comprising glucan extracted from diatoms. Said composition is preferably used in fish, shellfish, poultry, other animals and mammals

**Background of the invention**

Immunomodulatory compounds are attractive for the prophylactic treatment of cultivated fish and animals, to enhance the overall resistance against diseases caused by pathogenic microorganisms. The immunostimulants activate the non-specific immune system, by binding to macrophages. Positive effects from administrating non-specific immunostimulants to animals through the feed or water are increased growth, survival and disease resistance. Non-specific immunostimulants can also be administered to animals by injections, as components of vaccines. D'SOUZA F.M.L. ET AL.: "Flocculated microalgae concentrates as diets for larvae of the tiger prawn Penaeus monodon Fabricius" AQUACULTURE NUTRITION Vol. 8, no. 2, 2002, pages 113-120, describes the use of whole, living cells of different diatom species as the only feed for prawn larvae.

DE 196 08 563 A1 describes the use of a long range of microalgae or derivatives as additive in preparation of medicine or nutrient-additives in health food or tablets, together with a long range of plants and other compounds. Beta-glucans are mentioned as a cholesterol-lowering, blood-pressure-lowering and anti-carcinogenic compound found in the plant Ganodema lucidum, which is not a microalga.

RAA J. ET AL.: "The use of immune-stimulants in fish and shellfish feeds" AVANCES EN NUTRICION ACUICOLA V. MEMORIAS DEL V SIMPOSIUM INTERNACIONAL DE NUTRICION ACUICOLA 19 November 2000 - 22 November 2000, pages 47-56, describes the immunostimulatory effects of beta-1,3/1,6-glucans in animals and humans. The best documented immunomodulatory glucan is derived from yeast cell walls. Macrogard ® is a commercial yeast glucan that is used in fish feed etc. Microalgae are not mentioned as a potential source for immunomodulatory beta-1,3/1,6-glucans.

GRANUM E.: "Metabolism and function of Beta-1,3-glucan in marine diatoms" January 2002, DEPARTMENT OF BIOTECHNOLOGY, FACULTY OF NATURAL SCIENCES AND TECHNOLOGY, NORWEGIAN UNIVERSITY OF SCIENCE AND TECHNOLOGY (NTNU), TRONDHEIM pages I-VII, 1-48, PAPER 1-5 (URN:NBN: No-2101), describes that the principal storage product in diatoms (chrysolamin-aran) is a beta-1,3-D-glucan with occasional branching through C-2 and C-6.

Because the immunomodulatory compounds stimulate the non-specific immune system of the animal, non-specific immunostimulation can be used on early juvenile stages, before the specific immunesystem mature and the animal can be vaccinated with proper vaccines. This may be very valuable as an alternative to antibiotics in intensive juvenile production, especially in aquaculture and chicken production, but also for other animals and mammals where antibiotics are regularly in the use prophylactic for growth enhancement and for the reduced disease outbreak.

In intensive fish farming of marine species, cultivated live feed organisms such as rotifers and Artemia are used as feed for the larvae during the earliest stages. Cultivation of live feed induces growth of opportunistic microorganisms, and when rotifers and Artemia are fed to larvae, these bacteria and virus are transferred into the larvae and the rearing water. Intensive cultivation conditions may cause stress-induced immuno-suppression of the larvae, and opportunistic bacteria may therefore proliferate and harm the larvae. For instance larvae of intensively cultivated cod (*Gadus morhua L.*) and other species are vulnerable to stress and disease outbreak. Especially *Vibriosis* may kill large numbers of larvae and juveniles. The change from live food to formulated food is also a very critical stage in the first-feeding. The formulated diet may be of high quality both concerning nutritional value, digestibility and physical properties. However, the introduction of food particles into the fish tank and larval gut loads the substrate for bacterial growth. The loading of substrate for bacterial growth can also favour growth of fast growing, opportunistic bacteria, which may be harmful to the larvae. The larvae can therefore easily be heavy colonized and infected by bacteria. High mortality rates are therefore often observed during weaning phases. Methods to enhance the resistance of the larvae against bacterial infections by non-specific immuno-stimulation, can be very attractive. Beta-glucans are important structure-polysaccharides of the different yeasts and algae, and beta-glucans with 1-3 linkages and side chains linked to the main chain in 1-6 position are documented in vitro and in vivo to have immunomodulatory effects on fish and animals. Macrogard®, see EP 0466037, is a commercially available beta-glucan, prepared from baker's yeast, *Saccharomyces cerevisiae.* It is the overall leader as an immunostimulant on the fish feed market. It is used as a special ingredient in fish feed for salmonids and for marine fish and shellfish. The glucan in brown seaweed is denoted laminaran, and is a beta-1,6-branched beta-1,3-D-glucan. Laminaran from the macro algae *laminaria hyperborea* is documented to have immunomodulatory effect and enhances the disease resistance of fish species as Atlantic salmon (Dalmo, R.A. and Seljelid, R. 1995, The immunomodulatory effect of LPS, laminaran and sulfated laminaran [β(1,3)-D-glucan] on Atlantic salmon, Salmo salar L., macrophages in vitro. Journal of Fish Disease18, 175-185.) and blue gourami (Samuel, M., Lam, T. J. and Sin, Y. M. 1996. The effect of Laminaran [β(1,3)*D*Glucan] on the protective immunity of blue gourami, Trichogaster trichopterus against Aeromonas hydrophila. Fish & Shellfish Immunology 6, 443-454.). However, laminaran is not in use as an immunomodulating additive in commercial fish feed. Another algal polysaccharide with immunomodulating properties is High-M-alginate, containing 80-99% M-blocks. High-M-alginates are available commercially.

Beta-1,3-glucans are also important storage-polysaccharides in many classes of microalgae. These glucans are denoted chrysolaminaran, and are relatively short-chained (DP 20-60). Especially are diatoms (*Bacillariophyceae* sp.) known to accumulate large amounts of beta-glucans during the stationary growth phase. It is not documented earlier however, that chrysolaminaran has immunomodulating properties.

As stated above, it is well documented that several algal groups produce β-1,3-glucans, but this gives no reason to expect that all these β-1,3-glucans are immunomodulatory. Further, it is not possible to predict which of the algal species produces immunomodulatory β-1,3-glucans without testing. The β-1,3-glucans can vary between the different groups, species and according to the growth phase when the algae are harvested and the glucan extracted. Variations can be in length of the molecule and the degree of branching. Therefore, it can not be predicted which algal β-1,3-glucans will be the most optimal.

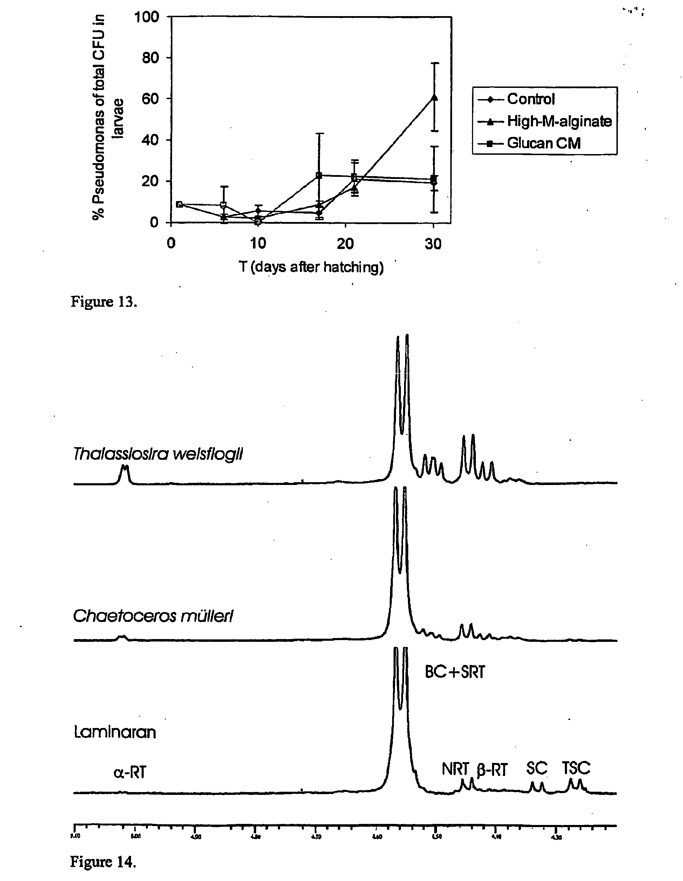

The structures of the different glucans are described using the degree of polymerization, DP, which gives the number of glucopyranosyl units in the molecules, and degree of branching, DB, which describes the ratio of β-(1→6)- to β-(1→3)-linkages. DP and DB are calculated from $^1$H-NMR spectra recorded of the glucans (Kim, Y. T. et al., Structural characterization of β-D-(1→3, 1→6)-linked glucans using NMR spectroscopy, 2000, Carbohydrate Research, 328: 331-341) by which Laminaran has been reported having a DP/DB ratio of, 33/0.07. Macrogard is a highly branched β-(1→3),(1→6)-glucan having a DP of 400-1500 (European Patent No. 0466037). The glucan isolated from the microalgae *Chaetoceros mülleri* and *Thalassiosira weissflogii* was determined to have DP/DB of 19-24/0.005-0.009 and 5-13/0, respectively. The number of β-(1→6)-branches per molecule in Laminaran is calculated to be 2.31, and for the *C. mülleri* glucan one branch per 5-11 chains. In Laminaran the branches consist of β-(1→3)-linked chains connected to the main chain by the β-(1→6) -branch point, whereas in the C. *mülleri* glucan the branches consist of one single β-(1→6)-linked glucopyranose unit as no resonances were found from the side chain β-(1→3)-linked units. Thus, *C. mülleri* glucan resembles the structure of laminaran in chain length, but not in degree of branching. The anomeric region of the $^1$H-NMR spectra obtained from the glucans isolated from C. *mülleri, T. weissflogii* and Laminaran are presented in Figure 14, which shows the differences in the structures. Figure 14 thus further shows that β-1,3-glucans from two different, centric diatoms, *Thalassiosira weissflogii* and *Chaetoceros mülleri* produce glucans that are structurally different. Further, as described below in experiment with cod larvae, these two β-1,3-glucans had completely different effects of the survival of larvae. These results document that the structure of the β-1,3-glucan has high impact on the immunomodulatory properties of the molecules, and that it is not obvious that all diatoms synthesize immunomodulatory β-1,3-glucans.

Laminaran, the glucan in brown algae, is extracted from algae that are harvested from the sea. Because the structure of algal polysaccharides in both micro- and macro-algae growing in the sea changes due to seasonal shifts in the growth conditions (light, temperature, nutrient availability, salinity, current), the immunomodulatory effect of the glucans can possibly vary between batches harvested at different time during the year, as well as between batches from different geographical localities. This, in addition to the fatiguing harvesting of algae from the sea, is a very large drawback when comparing to using chrysolaminaran as an immunostimulant, commercially. Besides, the harvesting of algae disturbs the local biological balance on the harvesting place. Harvesting of brown algae has thus great ecological impact on the seaweed populations, the sea bottom and side as biotope and on the seaweed as biotope for other species. Microalgae, on the other hand, can be cultivated industrially in photobioreactors, accurately adjusted for the optimal synthesis of the selected β-glucan. This makes an industrial production of a β-glucan with exact chemical structure possible at a whole year base, securing a stable availability of β-glucan of known quality for delivery to the feed- and pharmaceutic industry. Using crysolaminaran from cultivated microalgae, such as diatoms, is therefore very advantageous compared to laminaran from harvested brown algae.

There is a need in the present field to reduce or avoid the use of antibiotics in breeding and culturing of animals and mammals. Thus, it is an object of the present invention to provide a pharmaceutical composition, compound, feed and constituent of feed having immuno-stimulating, prophylactic and growth enhancing effects, in order to reduce the prophylactic and therapeutic use of antibiotics, especially in aqua culture and chicken production, but also for other animals and mammals.

## Summary of the invention

The present invention thus describes a pharmaceutical composition having immunostimulating, prophylactic and/or growth enhancing effects for use in enhancing the overall resistance against diseases, caused by pathogenic and opportunistic pathogenic micro-organisms, comprising glucan extracted from diatoms. In one embodiment said diatom is, from Bacillariophyta sp., as a *Chaetoceros milleri.* In a further embodiment the pharmaceutical composition comprises said diatoms are in dried form.

Further, aspect of the present invention is a compound for immunostimulation having prophylactic and/or growth enhancing effects for use in enhancing of the overall resistance against diseases caused by pathogenic and opportunistic pathogenic microorganisms, comprising glucan from diatoms extracted.

A feed having immunostimulating, prophylactic and/or growth enhancing effects for use in enhancing the overall resistance against diseases caused by pathogenic and opportunistic pathogenic microorganisms, comprising glucan extracted from diatoms, is also provided in the present invention. In one embodiment said feed comprises glucan extracted from diatoms in an amount that enhances the growth rate and/or disease resistance. In a further embodiment the feed comprises glucan extracted from diatoms. Further aspects of the present invention is a constituent of feed having immunostimulating, prophylactic and/or growth enhancing effects for use in enhancing the overall resistance against diseases caused by pathogenic and opportunistic pathogenic microorganisms, comprising glucan extracted from diatoms. In one further embodiment said constituent of feed comprises glucan extracted from diatoms and/or formulated feed.

A further aspect of the invention is use of a composition comprising glucan extracted from diatoms for the manufacture of a pharmaceutical composition having immunostimulating, prophylactic and/or growth enhancing effects for enhancing the overall resistance against diseases caused by pathogenic and opportunistic pathogenic microorganisms for the prophylactic treatment of fish, shellfish, poultry, other animals and mammals.

A further aspect of the invention is use of a composition comprising a glucan extracted from diatoms for producing a feed or constituent of feed for immunostimulation. prophylaxis and/or growth enhancement.

The invention further describes use of a composition comprising a glucan extracted from diatoms for the manufacture of a constituent or additive of feed for culturing of fish, shellfish, poultry, other animals and mammals.

## Brief description of the figures

Fig. 1    shows the survival of cod larvae treated with Glucan CM, Glucan TW or Macrogard® via rotifers, and in control, with average survival percentages for 3 replicates.

Fig. 2    shows the average survival of 27days old cod larvae treated with Glucan CM, Glucan TW or Macrogard® via rotifers, and in control. N = 3. * denotes statistical significant difference from control.

Fig. 3    shows the relative percent protection (RPP) from treatment of cod larvae with Glucan CM or Macrogard®.

Fig. 4    shows the growth of cod larvae treated with. Glucan CM or Macrogard®, and in control, measured as dry weight.

Fig. 5    presents the specific daily growth rates (SGR) for cod larvae treated with Glucan CM, Glucan TW or Macrogard® and in control during 4 periods of the experiment.

Fig. 6    shows growth of cod larvae treated with Glucan CM or High-M-alginate, and in control.

Fig. 7    shows growth of cod larvae treated with Glucan CM, Glucan TM or Macrogard® and in control.

Fig. 8    shows daily specific growth rates of cod larvae in period with first feeding and weaning to dry feed, where the cod larvae were treated with Glucan CM or High- M-alginate and in control.

Fig. 9    shows the bacterial numbers in cod larvae gut at day 27 after hatching, measured on M65 seawater agar (total CFU), *Pseudomonas Agar (Pseudomonas* sp.) and TCBS *(Vibrio* sp.), where the larvae were treated with Glucan CM, Glucan TM or Macrogard® and in control.

Fig. 10    shows the average survival of cod larvae during weaning to formulated food, wherein the larvae were fed control rotifers from days 2-18, and bioencapsulated Glucan CM or High-M-alginate were administrated on days 2-3, 9-11, 15-17 and 20-22.

Fig. 11    shows the total CFU in larval gut of cod larvae fed High-M-alginate or Glucan CM bioencapsulated in rotifers, and in control larvae.

Fig. 12    shows per cent *Vibrio* in larval gut measured on TCBS, the average of two tanks per treatment, wherein the cod larvae were treated with Glucan CM or High-M- alginate or fed only control rotifers.

Fig. 13    shows per cent marine *Pseudomonas* in larval gut measured on *Pseudomonas* CFC agar, the average of two tanks per treatment, wherein the cod larvae were treated with Glucan CM or High-M-alginate or fed only control rotifers.

Fig. 14    shows the anomeric region of the $^{1}$H-NMR spectra of the glucans from C. *mülleri* and *T. weissflogii;* α-RT: the α-reducing end resonance; BC: β-(1→3) backbone chain Resonance; SRT: Second to the reducing terminal group resonance; NRT: The non-reducing end group resonance; β-RT: the β-reducing end group resonance; SC: side chain group resonances, and TSC: terminal side chain resonances.

Fig. 15    shows total bacterial numbers measured as colony forming units on marine Agar, in tank water with cod larvae fed High-M-alginate or Glucan CM bioencapsulated in rotifers, and with control larvae.

**Detailed description of the invention**

The present invention describes a pharmaceutical composition having immunostimulating, prophylactic and/or growth enhancing effects for use in enhancing the overall resistance against diseases caused by pathogenic and opportunistic pathogenic microorganisms, comprising glucan extracted from diatoms.

Further, the glucan may be encapsulated in alginate microbeads. However, other forms of microbeads or encapsulation may be used. Encapsulation is, however, not necessary since the glucan may be administered as such.

The pharmaceutical composition according to the present invention, which is an immunostimulating agent, stimulates the non-specific immune system of the animal or non-human mammal. Thus, non-specific immunostimulation may be used on early juvenile stages before the specific inmune system matures. The animal or non-human mammal may thus be vaccinated with the pharmaceutical composition according to the present invention. It may be used for prophylactic and/or growth enhancing effects by enhancing the overall resistance against diseases caused by pathogenic and opportunistic pathogenic microorganisms.

Further, in fish farming is the change from feeding with live food to formulated food a critical stage in the first-feeding period and low survival is often observed. The resistance of the larvae against bacterial infection is significantly improved by the unspecific immunostimulation. The experiments below have shown more than 30% increased survival in the dried feed period The pharmaceutical composition may also be a part of formulated food used both for feeding of juvenile and adult stages of fish, shellfish, poultry, other animals and mammals. It may also be used in the weaning period for cod larvae, or for larvae from any other cultured marine species of fish and shellfish that have to be weaned from live food to dried diets. In the example section cod larvae are used, however, the invention should not be limited to cod or fish, as it most likely will be just as effective to other animals and mammals.

In an embodiment according to the present invention said diatom is *Bacillariophyceae* sp. However, other diatoms may also be effective.

The present invention further concerns a compound for immunostimulation having prophylactic and/or growth enhancing effects for use in enhancing of the overall resistance against diseases caused by pathogenic and opportunistic pathogenic microorganisms, comprising glucan extracted from diatoms.

In one embodiment is the compound according to the present invention encapsulated in alginate microbeads. However, other types of microbeads may be used with good effect. Further, the compound does not have to be encapsulated as it may be used as such. The compound according to the present invention may be added to the water that the animals and mammals recide in or ingest.

Since the compound according to the present application has immunomodulatory effect, it may be used as adjuvants in vaccines for treatment of animals and mammals.

Further, the compound may be administered to animals and mammals, as humans, in powder or tablet form as an immunomodulatory agent or a dietary supplement.

An aspect of the present invention concerns feed having immunostimulating, prophylactic and/or growth enhancing effects for use in enhancing the overall resistance against diseases caused by pathogenic and opportunistic pathogenic microorganisms, comprising micro-glucan extracted from diatoms.

In an embodiment according to the present invention the feed comprises glucan extracted from diatoms. Rotifers or other live feed organisms may be fed or treated with said feed. Further, the rotifers with bio-encapsulated feed may be offered to animals and other mammals.

In aquaculture it is possible to administer the glucan directly into water. Glucan can also be administered via the drinking water for poultry and mammals.

The present invention further describes a constituent of feed having immunostimulating, prophylactic and/or growth enhancing effects for use in enhancing the overall resistance against diseases caused by pathogenic and opportunistic pathogenic microorganisms, comprising glucan extracted from diatoms.

The present invention further comprises use of a composition comprising glucan extracted from diatoms for the manufacture of a pharmaceutical composition having immunostimulating, prophylactic and/or growth enhancing effects for enhancing the overall resistance against diseases caused by pathogenic and opportunistic pathogenic microorganisms for the prophylactic treatment offish, shellfish, poultry, other animals and non-human mammals.

Further, a glucan extracted from diatoms may be used for enhancing the overall resistance agains diseases caused by pathogenic and opportunistic pathogenic microorganisms. A composition comprising glucan extracted from diatoms may according to the invention further be used for producing a feed or constituent of feed for immunostimulation prophyl axis and/or growth enhancement according to the present invention.

The invention will in the following be described by examples. The examples are for illustrating purposes. Thus, in the examples cod larvae are used, but other marine fish species as salmon or other animals and mammals would show same effects.

**Experimental**

**Example 1**

*Treatment of cod larvae with chrysolaminaran from a culture of the diatom Chaetoceros mülleri*

Chrysolaminaran was extracted from a culture of the diatom *Chaetoceros mülleri* (this chrysolaminaran will from now on be called Glucan CM), incorporated in food for marine fish larvae and fed to cod larvae. Two other groups were fed Macrogard® (KS Biotec-Maczymal, Tromsø, Norway) and ordinary food. Effects on larval growth, survival and gut colonization were measured, and the results are given in the attached figures.

*C.mülleri* was cultivated in 200 liter f/2-medium (Guillard, R.RL. & Ryther, J.H. 1962. Studies of marine planktonic diatoms. I. Cyclotella nana Hustedt, and Detonula confervacea (Cleve) Gran. Canadian Journal of Microbiology 8, 229-239.), at 20°C and with continuos light of 70-90 $\mu$mol m$^{-2}$ s$^{-1}$. The growth medium was prepared from micro filtered (5 $\mu$m) seawater plus added nutrients, and the culture aerated with filtered air added <0,1 % $CO_2$. When the algal culture was at the stationary growth phase (6 days after inoculation of medium), the cells were harvested by centrifugation. Cellular beta-1,3-glucan was extracted by 0.0005 M H2S04 at 60°C. The extract was filtered on the Whatman GF/C filter, the filter was washed with distilled water and the extract was dialyzed (1000 MWCO) against Milli-Q water. The dialyzed extract was concentrated in a rotatory evaporator and freeze dried. The dried extract was stored in vials, at 4°C. The structure of the extract was characterized by $^1$H- and $^{13}$C-DEPT-NMR spectroscopy. By the method of Kim et al. above, the experimental results correspond to a beta-1,3-glucan, with a degree of polymerization of 19-24, corresponding to a molecular weight of ~4500-5200, and a degree of 1-6 branching of 0.003-0.005. 13C- DEPT-NMR confirmed the beta 1-3 structure of the glucan.

The Glucan CM was encapsulated in alginate microbeads (Skjermo, J. et al., (1995)). Immunostimulation of juvenile turbot (Scophthalmus maximus L.) using an alginate with high mannuronic acid content administered by the live food organism Artemia. (Fish & Shellfish Immunology 5, 531-534), by mixing 1 g glucan and 3 g LF 10/60 alginate (FMC Biopolymer, Drammen, Norway) into 360 ml distilled water and spraying the solution through a nozzle into an aqueous solution of calcium chloride (50 mM) and methanol (5%). The micro beads were harvested on a sieve, washed and stored in water containing 0.1% formaldehyde.

Eggs from cod were placed in 701's tanks with a flat bottom and central outlet. The water inlet was through tubes near the tank wall, perforated to create a circular water current in the tank. The start density was 100 eggs per liter. The algal paste of Nanocloropsis oculata (Reed Mariculture) was added to the tank water from day 1 after hatching. The larvae were fed rotifers cultivated with yeast and a marine oil from day 2 after hatching. From day 20 the larvae were offered formulated dry food. The experiment was terminated on day 27 after hatching.

The Glucan CM microbeads and Macrogard® were bioencapsulated in rotifers (*Brachionus plicatilis),* by incubating the rotifers for 20-30 min in densities of 250 ind ml$^{-1}$ in seawater added 0,5 gl$^{-1}$ of the immunostimulants. The treated rotifers were fed to cod larvae as three meals per day on days 4, 11 and 18 after hatching. The control group was fed rotifers cultivated with yeast and marine oil at these meals. The experiment was run with 3 replicates per treatment, in total nine tanks. The first treatment with immunostimulants, at day 4 after hatching, was chosen because it was expected that the larvae needed a couple of days to start ingesting rotifers at optimal rates.

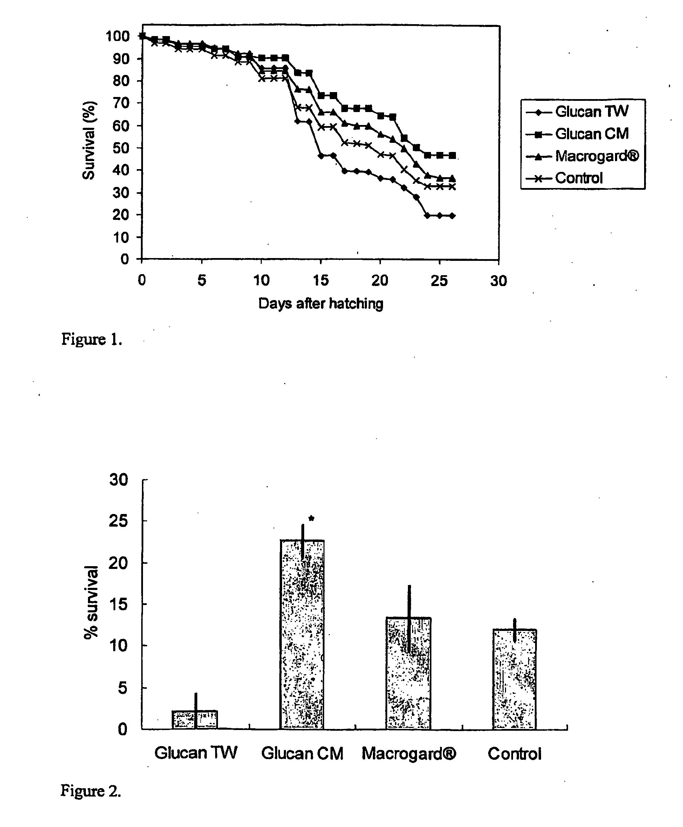

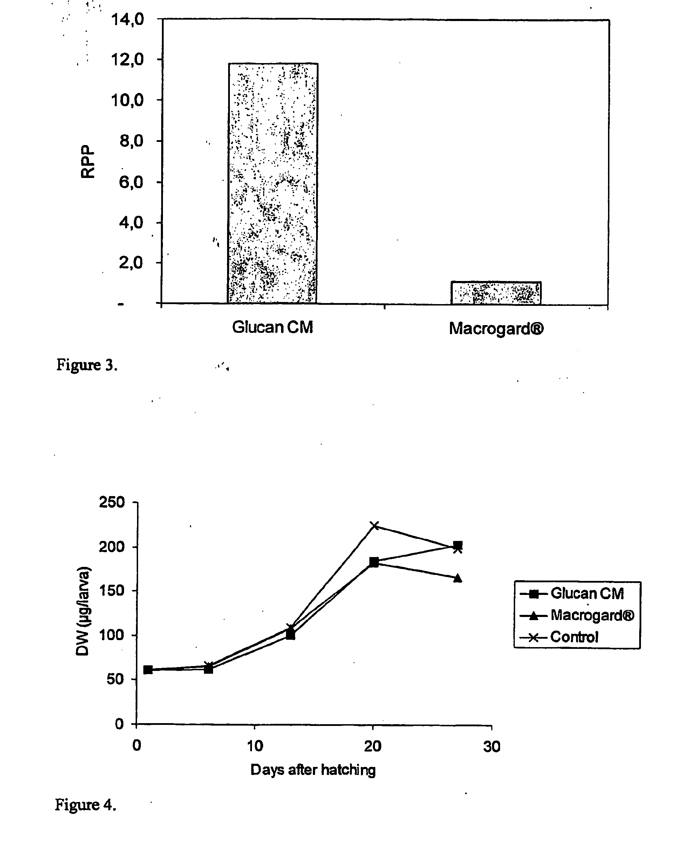

Samples for determination of the larval growth were collected on days 1, 6, 13, 20 and 27 after hatching. Survival was determined every second and third day, by siphoning the bottoms and counting dead larvae. After introduction of the formulated feed, siphoning was performed daily. From day 10 after hatching the survival of the cod larvae was highest in the group fed Glucan CM (see Figure 1). The total survival of the Glucan CM-group was increased by respectively, 41 and 47% compared to the groups fed Macrogard® and the control at day 27 (see Figure 2). This indicates a 10 times higher Relative Percent Protection for the group fed Glucan CM compared to the groups fed Macrogard® (see Figure 3). The Coefficients of Variance (CV) of the survival percentages were 14, 51 and 19% for groups treated with Glucan CM, Macrogard® and control, respectively. This indicates that the reproducibility of the survival within the treatments was also increased by feeding the larvae Glucan CM, compared to Macrogard®. The poor reproducibility in the Macrogard® indicates that this treatment had no positive or even negative effects on the cod larvae in this experiment, whereas the good reproducibility between the control tanks indicates that the experimental set up functioned well.

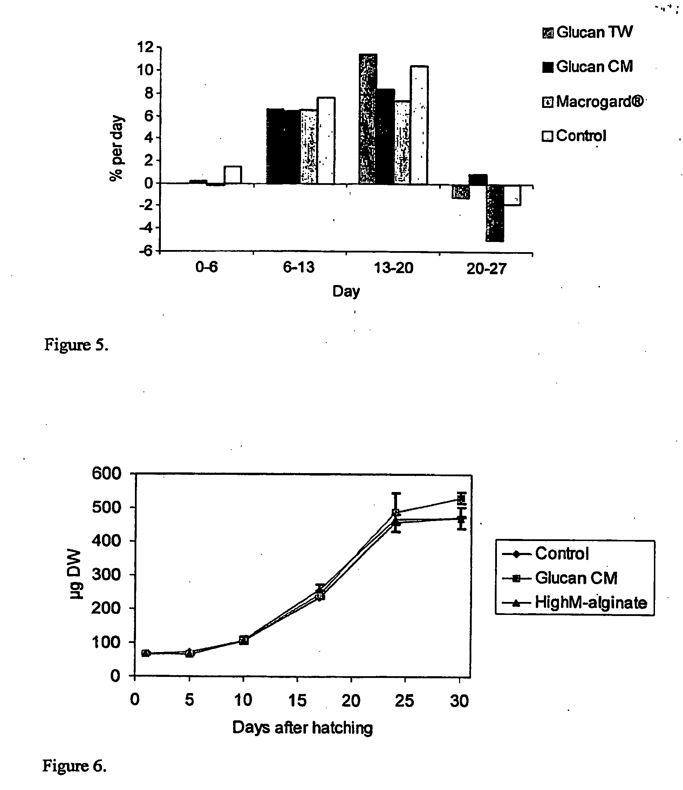

After one week feeding on the formulated diet, both the Macrogard® and control groups had reduced dry weights (see Figure 4). The negative slope from days 20 to 27 is due to increased mortality of the biggest larvae. However, the group fed Glucan CM continued to grow also after the changing from rotifers to the formulated diet, and reached higher larval weights than the Macrogard® group. Further, the specific growth rate in the period with the formulated diet (days 20-27) was positive only for the group that had been fed Glucan CM during the rotifers' feeding periods (see Figure 5).

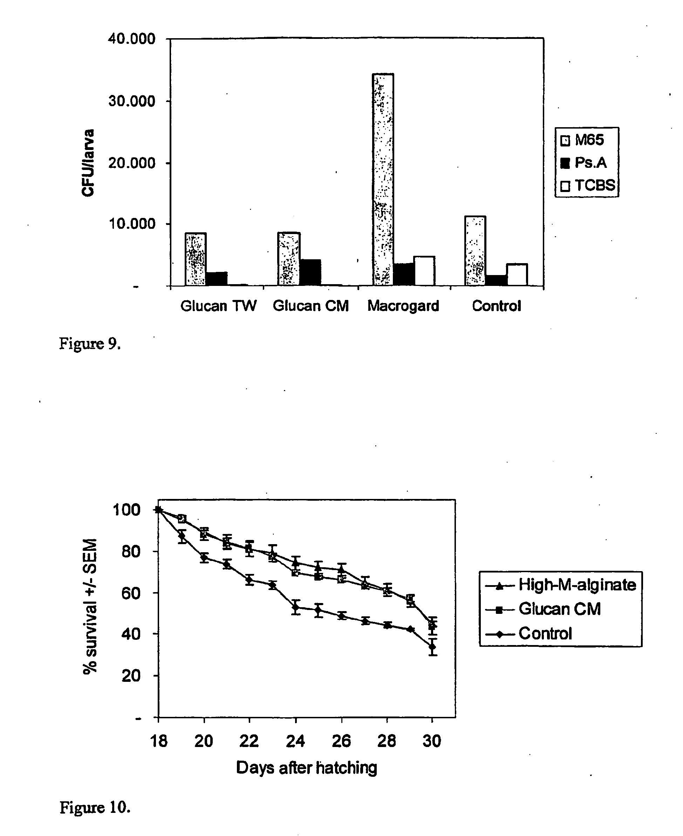

The larvae treated with Glucan CM had significantly lower numbers of bacteria in the gut (see Figure 9). The control larvae and the group treated with Macrogard® had respectively, 11 000 and 22 000 total CFU in the guts, corresponding to 30 and 156% higher bacterial content than in the Glucan CM larvae. The Glucan CM group also had very low content of *Vibrio* bacteria (200 per larva), measured on TCBS agar, compared to the control and the Macrogard® groups, that contained 7000 and 4600 *Vibrio* CFU per larva, respectively. In the Glucan CM larvae the *Vibrio* sp. corresponded to 2% of the total CFU, whereas *Vibrio* sp. accounted for 63 and 21% of the total bacterial numbers in control and Macrogard® treated larvae, respectively. The number of *Pseudomonas* sp. was not abnormally high in any groups.

Beta-1,3-glucans are known to be immunomodulatory, and the positive effects obtained from treating cod larvae with beta-1,3-glucan from a marine diatom indicate that this glucan has strong immunomodulating properties. The immunostimulating treatment hence prepared the larvae to better tolerance against the bacterial stress introduced by the change of diet. This was confirmed by the much lower bacterial numbers that were quantified in the larvae treated with Glucan CM, than in control larvae and larvae treated with glucan from Saccharomyces cerevisiae (Macrogard®).

The very good resistance against colonization by *Vibrio* sp. in the Glucan CM treated group is also expected to be a direct result from the immunostimulation, and is a very positive effect. Among the *Vibrios* there are many pathogenic species to fish, both opportunistic and obligate, and outbreak of Vibriosis following stress is a well-known problem in the juvenile production of cod. High numbers of *Vibrio* sp. in the cod larvae at day 27 therefore suggest the poor condition of larvae and possible infections. For the Glucan CM treated group it is suggested that the glucan was bound to receptors on macrophages in the gut mucosae of the cod larvae. Thereby the non-specific immune system in the gut of the larvae was stimulated to better resistance against bacterial colonization and infection, possibly from *Vibrio* sp.

Today's first feeding regimes for cod larvae normally includes the feeding with Artemia from around days 17-20 and for 2-3 weeks, before the larvae are weaned to formulate dry food. In this experiment it was shown that immunostimulation of cod larvae with Glucan CM during the rotifer period permitted a very early weaning of the larvae, because the larvae had acquired a better tolerance to the bacterial stress induced by the formulated food.

The glucan from marine diatoms has a better immunomodulating effect on cod larvae than the commercially available yeast glucan Macrogard®, which had no improving effects on the larval viability. The cod larvae fed Macrogard® were considerably colonized in the gut by *Vibrio.* The difference between the glucans from the marine diatom (Glucan CM) and from yeast cell walls (Macrogard®) is mainly the water solubility and degree of branching. The positive effects from Glucan CM on cod larvae may be due to this difference. The Glucan CM molecule may have a structure that makes it more suitable for binding to receptors on the macrophages, thereby inducing non-specific immune responses such as enhanced phagocytosis and production of oxidative radicals more quickly than Macrogard®. Huge advantages may thus be achieved by using glucan from marine diatoms, instead of glucan from yeast.

**Example 2**

*Treatment of cod larvae with Glucan TW, Glucan CM, Macrogard® and control rotifers*

β1,3-glucan from the microalgae *T. weissflogii,* Glucan TW, was produced, isolated and administrated to cod larvae using the same methods as for Glucan CM in Example 1. Glucan TW, Glucan CM and Macrogard ® were thus administered via the feed to cod larvae.

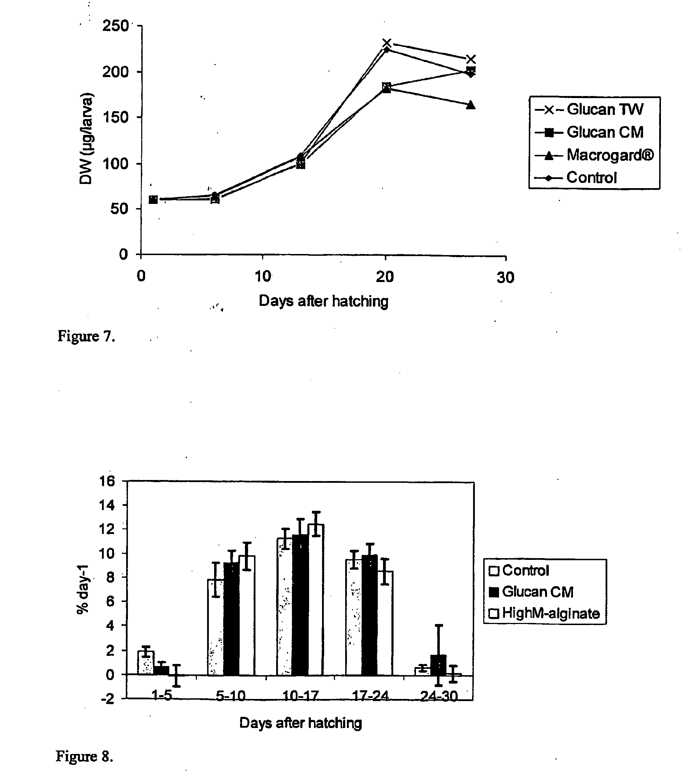

As shown in Figure 7, cod larvae treated with Glucan TW had high growth rate from days 13-20, but after introduction of formulated dry feed, the growth stopped. Only cod larvae treated with Glucan CM continued to grow in the period with dry feed. Structural studies of Glucan CM and Glucan TW has shown that these two different centric diatoms produce glucans that are structurally different, see Figure 14. This experiment has shown that these two β-1,3-glucans have completely different effects on larvae survival. These results thus document that the structure of the β-1,3-glucan has high impact on the immunomodulatory properties of the molecules.

**Example 3**

*Treatment of cod larvae with Chrysolaminaran (Glucan CM) and High-M-alginate*

**Assay**

Glucan CM was extracted from a culture of the diatom *Chaetoceros mülleri,* incorporated in food for marine fish as stated in above Example 1 and fed to cod larvae. Two other groups were fed with High-M-alginates and ordinary food.

The effects on larval growth, survival and gut colonization were measured.

The assay was run with 3 replicates per treatment in total nine tanks, with the application of standard conditions as stated in Table 1. Thus 3 triplicates were used for treatment with High-M-alginate, Glucan CM and untreated control. 150 eggs per liter from cod were placed into the tanks 3 days before hatching. The larvae were from days 3-24 after hatching fed cultivated and enriched rotifers. From days 18-30 after hatching the larvae were fed formulated dry food. Further, algal paste (of *Nannochloropsis oculata*) were also offered from day 3 after hatching and throughout the period when larvae were fed rotifers.

High-M-alginate and Glucan CM were encapsulated in rotifers and fed the larvae on days 3-4, 9-11, 15-17 and 20-22. The control group received only ordinary cultivated and enriched rotifers.

**Table 1**

**Sandard conditions for firstfeeding of cod larvae**

| | |
|---|---|
| Eggs from cod | Start density 100 larvae liter$^{-1}$. |
| Tanks for assays | 150 liter, with central outlet. |
| Quality of water | Seawater from 90 m depth, sand filtered, tempered and aired. 34 % salt content. |
| Change of water | Days 0-2: 1 day$^{-1}$ |
| | Days 3-5: 2 day$^{-1}$ |
| | Days f-8: 3 day$^{-1}$ |
| | Days 9-12: 4 day$^{-1}$ |
| | Days 13-15: 6 day$^{-1}$ |
| | Days 16-30: 8 day$^{-1}$ |
| Temperature of water | Gradual increase from 8°C to 12°C from days 3-6. The temperature was maintained at $12 \pm 0,2$°C. |
| Light | Continuously from days 1-30. |
| Algal paste (*Nanocloropsis oculata*) | Algal paste was give 1-3 times per 24 h from days 3-25 (1,5-4 ml tank$^{-1}$). |
| Rotifers (*Brachionus pilcatilis*) | Rotifers were grown in tanks of 300 liters Daily dilution of 20% water with 20‰ salt content and temperature of 20°C. The rotifers were grown on yeast, Marol E and algal paste. Feeding; see Table 2. |
| Immunostimulation | High-M-alginate and Glucan CM, see Table 2. |
| Dry food | Feeding; see Table 2. |

**Feeding regime**

On day 3 the cod larvae were fed 5000 rotifers (*Brachionus pilcatilis*) grown on yeast Marol E and algal paste (*Nanochloropsis oculata*) liter$^{-1}$. On day 4 the larvae were fed morning and evening 5000 and 3000 rotifers liter$^{-1}$, respectively, and on day 5 were 3000 rotifers liter$^{-1}$ fed both morning and evening. From day 6 to day 20 the larvae were fed 3 times per day, and on days 21 and 22 the larvae were fed only morning and evening. The feed varied from 2000-7000 rotifers liter$^{-1}$ as required. On days 23 and 24, when the larvae are weaned to formulated dry food, they were fed 7000 rotifers liter$^{-1}$ in the evening. 2-4 ml algal paste (*Nanochloropsis oculata*) were added to the tanks morning and evening from day 3 to day 8, thereafter 3 ml morning and evening and 5,1 ml in the middle of the day from day 9 to day 20, 3 ml morning and evening from day 21 to day 24 and 3 ml in the morning on day 25.

Formulated dry food was added to the tanks from day 18 to day 30. In the daytime the larvae were further fed 1 g per 24 h on days 18 and 19 and 3 g per 24 h from day 20 to day 30.

## Table 2
## Firstfeeding of cod larvae

| Day | -2 | -1 | 0 | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|---|---|---|---|---|---|---|---|---|---|---|---|---|---|---|---|---|---|---|
| Immunostimulants (A,B) | | | | | | | Algal paste | | | | | | | | | | | | | | | | | | | | | | | | | | |
| | | | | | | | Rotifers | | | | | | | | | | | | | | | | Formulated feed | | | | | | | | | | |
| Control (K) | | | | | | | Algal paste | | | | | | | | | | | | | | | | | | | | | | | | | | |
| | | | | | | | Rotifers | | | | | | | | | | | | | | | | Formulated feed | | | | | | | | | | |
| Light | | | | | | | | | | | | | 24 hours light | | | | | | | | | | | | | | | | | | | | |
| Water exchange pr.d | 1x | | | | 2-3x | | | | | | | 6x | | | | | 8x | | | | | | | | | | | | | | | | |
| Temp. | 7,5 | | 8-12 | | | | | | | | | | 12 | | | | | | | | | | | | | | | | | | | | |
| Air | | | | | | | | | | | | | | | | | | | | | | | | | | | | | | | | | Air |
| Samplings | | | | | | | | | | | | | | | | | | | | | | | | | | | | | | | | | |
| C/N | X | | | | | | | | | | | | | | | | | | | | | | | | | | | | | | | X | |
| Histology | | X | | | | X | | | | | | | | | | X | | | | | | | | | | | | | | | | X | |
| Microbiology | X | | X | | X | X | | | | X | | | | | | X | | | | | | | | | | | | | | | X | | X |
| In situ hybridisation | X | | X | X | X | | | | X | | X | | | | | X | | X | | | | | | | | | | | | | | | X |
| Realtime PCR | X | | X | X | X | | | | X | | X | | | | | X | | X | | | | | | | | | | | | | | | X |

### Analysis

Samples for *in situ* hybridizing, RNA analysis, microbiology, C/N (dry weight and growth rate) and histolopathology was taken as shown in Table 2.

The dry weight is estimated based on the equation:

$$\mu g\ dry\ weight = 2{,}34 \times \mu g\ carbon$$

Samples for C/N analysis were taken on days XX (10-15 larvae).

The specific rate of growth (SGR, % day $^{-1}$) was estimated from the equation:

$$SGR = ln\ (N/N_l) \times 100/h,$$

wherein No is initial dry weight and $N_t$ is individual dry weight by time h. The equation is used for estimating the growth in the different parts of the assay, and with average weight for each tank as No for each period.

### Results

The survival was determined by counting the number of dead larvae and is shown in Figure 10.

The survival of the Glucan CM group and High-M-alginate group was respectively 43,9% and 45% on day 30 after hatching, and did not differ statistically during the experiment (p>0.05, Student's t-test)). The survival of the control group was, however, only 33,8%. From day 22 to 29 (the dry feed period) the mortality in the control group was statistically significant higher than in the two stimulated groups (p<0.05). The difference in survival between the immunostimulated groups and the control group was already registered under weaning on dry food and further in the period with reduced co-feeding. The mortality was specially high in the control group from days 18-20 and from days 23-24 compared with the groups treated with immunostimulants. From days 18-20 under weaning to dry feed the mortality was 23,1% in the control group and 10,8% and 11,8% in groups treated respectively with High-M-alginate and Glucan CM. On day 25, when the period with co-feeding with rotifers were terminated the mean survival was 72,1% and 67,5% in groups treated with High-M-alginate and Glucan CM, respectively, and the survival of the control group was only 51,3%.

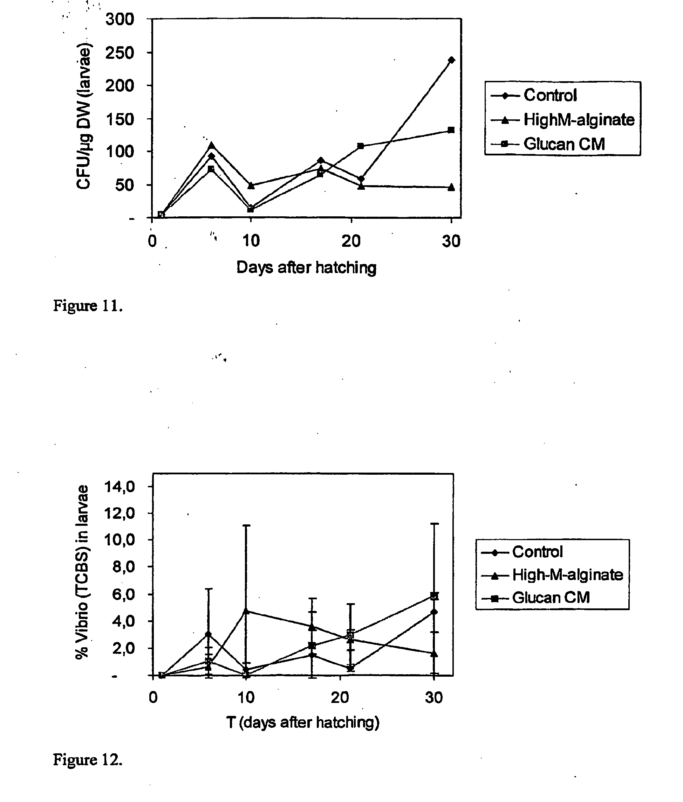

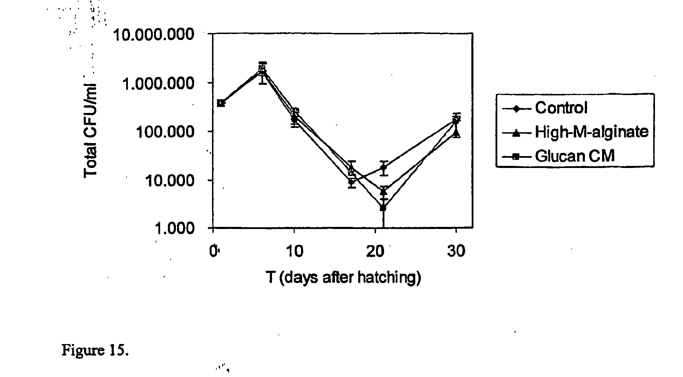

Further, the number of bacteria in the gut was analyzed. Samples were taken from all tanks on days 1, 6, 16 and 30. The larvae treated with the immunostimulants had a significantly lower number of bacteria in the gut compared to the control groups in the dry feed period, as shown in Figure 11. In the stimulated groups the increase was moderate from day 21 to 30, with 24% increase in the Glucan CM group and 3% in the High-M-alginate group. In the control group the bacterial content increased with 321% in this period, and at the same time as there was an increase in the total bacterial numbers in the tank water (Figure 15), caused by the introduction of dry feed. The immunostimulating treatment hence prepared the larvae to better tolerance against the bacterial stress introduced by the change of diet.

Figure 12 shows percentage *Vibrio* in the larval gut. The content of *Vibrio* varied among 0,4-5,9%, and increased slightly during the experiment. Figure 13 shows percentage marine *Pseudomonas* in the larval gut. The percentage of *Pseudomonas* did not increase in the Glucan CM groups and control groups after introduction to dry feed, but in the High-M-alginate group a marked increase of *Pseudomonas* was observed from days 21-30. The larval growth was measured and the dry weights are shown in Figure 6. The Glucan

CM group had the highest growth rates with larvae of 531 (30) $\mu$g. In the High-M-alginate group the larval weight was 470 (54) $\mu$g and in the control group 473 (6) $\mu$g. The larvae had the highest growth rate from days 10-17, but after introduction of dry feed the growth rate decreased significantly as shown in Figure 8. In the period with feeding of rotifers the High-M-alginate group had the best growth rate, but the Glucan CM group had the highest growth rate in the dry feeding period and obtained statistic significantly higher weights ($p < 0.05$).

**Example 4**

*Preparation of dried microalagae with high glucan content and production of formulated feed.*

*C. mülleri* is cultivated in 200 litre f/2-medium and harvested in the stationary growth phase by centrifugation. The algal paste is immediately freeze dried in a vacuum freeze drier. The dried micro algae are stored dark at -20°C. To produce formulated fish feed, the dried algae are added to the raw feed material mixture at two different concentrations, 5 and 50 grams per kilo, prior to extrusion of feed pellets. Control diet is made from the same mixture, but without algal addition. The feed is stored at -20°C prior to use.

The change from feeding with rotifers to feeding with dry feed is a very critical phase in the firstfeeding period and low survival is often observed in weaning of marine fish larvae. The resistance of the larvae against bacterial infections is significantly improved by the unspecific immunostimulation. The experiments showed that immunostimulation induced more than 30% increased survival in the dry feed period and also higher weight. Thus, it is shown that $\beta$-1,3-glucan from diatoms enhance the survival of cod larvae in a stressing phase of the first-feeding period, and that the larvae grow better and obtain higher weight.

**Claims**

1.   Pharmaceutical composition having immunostimulating, prophylactic and/or growth enhancing effects for use in enhancing the overall resistance against diseases caused by pathogenic and opportunistic pathogenic microorganisms, comprising glucan extracted from diatoms.

2.   Pharmaceutical composition for use according to claim 1, wherein said diatom is a Bacillariophyta sp.

3.   Pharmaceutical composition for use according to claim 2, wherein said Bacillariophyta sp. is a Chaetoceros mülleri.

4.   Pharmaceutical composition for use according to claims 1-3 wherein said glucan is a beta-1,3-glucan possessing 1-6 branching.

5.   Pharmaceutical composition for use according to claims 1-4 wherein said diatoms are in dried form.

6.   Compound for immunostimulation having prophylactic and/or growth enhancing effects for use in enhancing of the overall resistance against diseases caused by pathogenic and opportunistic pathogenic microorganisms, comprising glucan extracted from diatoms.

7.   Compound for use according to claim 6, wherein said diatom is a Bacillariophyta sp.

8.   Compound for use according to claim 7, wherein said Bacillariophyta sp. is a Chaetoceros mülleri.

9.   Compound for use according to claims 6-8, wherein said glucan is a beta-1,3-glucan possessing 1-6 branching.

**10.** Compound for use according to claims 6-9, wherein said diatoms are in dried form.

**11.** Feed having immunostimulating, prophylactic and/or growth enhancing effects for use in enhancing the overall resistance against diseases caused by pathogenic and opportunistic pathogenic microorganisms, comprising glucan extracted from diatoms.

**12.** Feed for use according to claim 11, wherein said diatom is a Bacillariophyta sp.

**13.** Feed for use according to claim 12, wherein said Bacillariophyta sp. is a Chaetoceros mülleri.

**14.** Feed for use according to claims 11-13 comprising glucan extracted from diatoms in an amount that enhances the growth rate and/or disease resistance.

**15.** Feed for use according to claims 11-14, wherein said diatoms are in dried form.

**16.** Constituent of feed having immunostimulating, prophylactic and/or growth enhancing effects for use in enhancing the overall resistance against diseases caused by pathogenic and opportunistic pathogenic microorganisms comprising glucan extracted from diatoms.

**17.** Constituent of feed for use according to claim 16, wherein said diatom is a Bacillariophyta sp.

**18.** Constituent of feed for use according to claim 17, wherein said Bacillariophyta sp. is a Chaetoceros mülleri.

**19.** Constituent of feed for use according to claims 16-18, wherein said diatoms are in dried form.

**20.** Use of a composition comprising glucan extracted from diatoms for the manufacture of a pharmaceutical composition having immunostimulating, prophylactic and/or growth enhancing effects for enhancing the overall resistance against diseases caused by pathogenic and opportunistic pathogenic microorganisms for the prophylactic treatment of fish, shellfish, poultry, other animals and mammals.

**21.** Use of a composition comprising a glucan extracted from diatoms for producing a feed or constituent of feed for immunostimulation prophylaxis and/or growth enhancement.

**22.** Use of a composition comprising a glucan extracted from diatoms for the manufacture of a constituent or additive of feed for culturing of fish, shellfish, poultry, other animals and mammals.

**23.** Use according to claim 20, wherein said composition comprising glucan is extracted from diatoms in dried form.

**24.** Use according to claim 23, wherein said diatom is a Bacillariophyta sp.

**25.** Use according to claim 24, wherein said Bacillariophyta sp. is a Chaetoceros mülleri.

**Patentansprüche**

**1.** Pharmazeutische Zusammensetzung, die immunstimulierende, prophylaktische und/oder wachstumsfördernde Wirkungen hat, zur Verwendung für die Verbesserung der Gesamtwiderstandsfähigkeit gegen Krankheiten, die durch pathogene und opportunistische pathogene Mikroorganismen verursacht werden, die Glucan enthält, das aus Diatomeen gewonnen wurde.

**2.** Pharmazeutische Zusammensetzung zur Verwendung nach Anspruch 1, wobei die Diatomee eine Bacillariophyta-Art ist.

**3.** Pharmazeutische Zusammensetzung zur Verwendung nach Anspruch 2, wobei die Bacillariophyta-Art eine Chaetoceros mülleri ist.

**4.** Pharmazeutische Zusammensetzung zur Verwendung nach den Ansprüchen 1 bis 3, wobei das Glucan ein beta-1,3-Glucan ist, das 1-6-Verzweigung(en) aufweist.

5. Pharmazeutische Zusammensetzung zur Verwendung nach den Ansprüchen 1 bis 4, wobei die Diatomeen in getrockneter Form vorliegen.

6. Verbindung für die Immunstimulation, die prophylaktische und/oder wachstumsfördernde Wirkungen hat, zur Verwendung für die Verbesserung der Gesamtwiderstandsfähigkeit gegen Krankheiten, die durch pathogene und opportunistische pathogene Mikroorganismen verursacht werden, die Glucan enthält, das aus Diatomeen gewonnen wurde.

7. Verbindung zu Verwendung nach Anspruch 6, wobei die Diatomee eine Bacillariophyta-Art ist.

8. Verbindung zur Verwendung nach Anspruch 7, wobei die Bacillariophyta-Art eine Chaetoceros mülleri ist.

9. Verbindung zur Verwendung nach den Ansprüchen 6 bis 8, wobei das Glucan ein beta-1,3-Glucan ist, das 1-6-Verzweigung(en) aufweist.

10. Verbindung zur Verwendung nach den Ansprüchen 6 bis 9, wobei die Diatomeen in getrockneter Form vorliegen.

11. Futter, das immunstimulierende, prophylaktische und/oder wachstumsfördernde Wirkungen hat, zur Verwendung für die Verbesserung der Gesamtwiderstandsfähigkeit gegen Krankheiten, die durch pathogene und opportunistische pathogene Mikroorganismen verursacht werden, das Glucan enthält, das aus Diatomeen gewonnen wurde.

12. Futter zu Verwendung nach Anspruch 11, wobei die Diatomee eine Bacillariophyta-Art ist.

13. Futter zur Verwendung nach Anspruch 12, wobei die Bacillariophyta-Art eine Chaetoceros mülleri ist.

14. Futter zur Verwendung nach den Ansprüchen 11 bis 13, das Glucan, das aus Diatomeen gewonnen wurde, in einer Menge enthält, die die Wachstumsgeschwindigkeit und/oder die Widerstandsfähigkeit gegen Krankheiten verbessert.

15. Futter zur Verwendung nach den Ansprüchen 11 bis 14, wobei die Diatomeen in getrockneter Form vorliegen.

16. Bestandteil eines Futters, der immunstimulierende, prophylaktische und/oder wachstumsfördernde Wirkungen hat, zur Verwendung für die Verbesserung der Gesamtwiderstandsfähigkeit gegen Krankheiten, die durch pathogene und opportunistische pathogene Mikroorganismen verursacht werden, der Glucan enthält, das aus Diatomeen gewonnen wurde.

17. Bestandteil eines Futters zur Verwendung nach Anspruch 16, wobei die Diatomee eine Bacillariophyta-Art ist.

18. Bestandteil eines Futters zur Verwendung nach Anspruch 17, wobei die Bacillariophyta-Art eine Chaetoceros mülleri ist.

19. Bestandteil eines Futters zur Verwendung nach den Ansprüchen 16 bis 18, wobei die Diatomeen in getrockneter Form vorliegen.

20. Verwendung einer Zusammensetzung, die Glucan enthält, das aus Diatomeen gewonnen wurde, für die Herstellung einer pharmazeutischen Zusammensetzung, die immunstimulierende, prophylaktische und/oder wachstumsfördernde Wirkungen hat, für die Verbesserung der Gesamtwiderstandsfähigkeit gegen Krankheiten, die durch pathogene und opportunistische pathogene Mikroorganismen verursacht werden, für die prophylaktische Behandlung von Fischen, Krustentieren, Geflügel, anderer Tiere und Säugetiere.

21. Verwendung einer Zusammensetzung, die ein Glucan enthält, das aus Diatomeen gewonnen wurde, für die Herstellung eines Futters oder eines Bestandteils eines Futters für die Immunstimulation, die Prophylaxe und/oder die Wachstumsförderung.

22. Verwendung einer Zusammensetzung, die ein Glucan enthält, das aus Diatomeen gewonnen wurde, für die Herstellung eines Bestandteils oder eines Zusatzes eines Futters für die Aufzucht von Fischen, Krustentieren, Geflügel, anderer Tiere und Säugetieren.

**23.** Verwendung nach Anspruch 20, wobei die Zusammensetzung, die Glucan enthält, aus Diatomeen in getrockneter Form gewonnen wird.

**24.** Verwendung nach Anspruch 23, wobei die Diatomee eine Bacillariophyta-Art ist.

**25.** Verwendung nach Anspruch 24, wobei die Bacillariophyta-Art eine Chaetoceros mülleri ist.


**Revendications**

**1.** Composition pharmaceutique ayant des effets immunostimulateurs, prophylactiques, et/ou de stimulation de croissance, destinée à être utilisée dans l'augmentation de la résistance totale à des maladies provoquées par des microorganismes pathogènes et des microorganismes pathogènes opportunistes, comprenant du glucane extrait de diatomées.

**2.** Composition pharmaceutique destinée à être utilisée suivant la revendication 1, dans laquelle ladite diatomée est une Bacillariophyta sp.

**3.** Composition pharmaceutique destinée à être utilisée suivant la revendication 2, dans laquelle ladite Bacillariophyta sp. est un Chaetoceros mulleri.

**4.** Composition pharmaceutique destinée à être utilisée suivant les revendications 1 à 3, dans laquelle ledit glucane est un bêta-1,3-glucane possédant une ramification 1-6.

**5.** Composition pharmaceutique destinée à être utilisée suivant les revendications 1 à 4, dans laquelle lesdites diatomées sont sous forme déshydratées.

**6.** Composé pour immunostimulation ayant des effets prophylactiques et/ou de stimulation de croissance destiné à être utilisé dans l'augmentation de la résistance totale à des maladies provoquées par des microorganismes pathogènes et des microorganismes pathogène opportunistes, comprenant du glucane extrait de diatomées.

**7.** Composé destiné à être utilisé suivant la revendication 6, dans lequel ladite diatomée est une Bacillariophyta sp.

**8.** Composé destiné à être utilisé suivant la revendication 7, dans lequel ladite Bacillariophyta sp est un Chaetoceros mulleri.

**9.** Composé destiné à être utilisé suivant les revendications 6 à 8, dans lequel ledit glucane est un bêta-1,3-glucane possédant une ramification 1-6.

**10.** Composé destiné à être utilisé suivant les revendications 6 à 9, dans lequel lesdites diatomées sont sous forme déshydratée.

**11.** Aliment pour animaux, ayant des effets immunostimulateurs, prophylactiques et/ou de stimulation de croissance, destiné à être utilisé dans l'augmentation de la résistance totale à des maladies provoquées par des organismes pathogènes et des microorganismes pathogènes opportunistes, comprenant du glucane extrait de diatomées.

**12.** Aliment pour animaux destiné à être utilisé suivant la revendication 11, dans lequel ladite diatomée est une Bacillariophyta sp.

**13.** Aliment pour animaux destiné à être utilisé suivant la revendication 12, dans lequel ladite Bacillariophyta sp est un Chaetoceros mulleri.

**14.** Aliment pour animaux destiné à être utilisé suivant les revendications 11 à 13, comprenant du glucane extrait de diatomées en une quantité qui augmente la vitesse de croissance et/ou la résistance aux maladies.

**15.** Aliment pour animaux destiné à être utilisé suivant les revendications 11 à 14, dans lequel lesdites diatomées sont sous forme déshydratée.

**16.** Constituant d'un aliment pour animaux, ayant des effets immunostimulateurs, prophylactiques et/ou de stimulation de croissance, destiné à être utilisé dans l'augmentation de la résistance totale à des maladies provoquées par des organismes pathogènes et des microorganismes pathogènes opportunistes, comprenant du glucane extrait de diatomées.

**17.** Constituant d'un aliment pour animaux destiné à être utilisé suivant la revendication 16, dans lequel ladite diatomée est une Bacillariophyta sp.

**18.** Constituant d'un aliment pour animaux destiné à être utilisé suivant la revendication 17, dans lequel ladite Bacillariophyta sp est un Chaetoceros mulleri.

**19.** Constituant d'un aliment pour animaux destiné à être utilisé suivant les revendications 16 à 18, dans lequel lesdites diatomées sont sous forme déshydratée.

**20.** Utilisation d'une composition comprenant du glucane extrait de diatomées pour la production d'une composition pharmaceutique ayant des effets immunostimulateurs, prophylactiques et/ou de stimulation de croissance pour augmenter la résistance totale à des maladies provoquées par des microorganismes pathogènes et des microorganismes pathogènes opportunistes pour le traitement prophylactiques de poissons, de mollusques et crustacés, de la volaille, d'autres animaux et de mammifères.

**21.** Utilisation d'une composition comprenant un glucane extrait de diatomées pour la production d'un aliment pour animaux ou constituant d'un aliment pour animaux à des fins d'immunostimulation, de prophylaxie et/ou de stimulation de croissance.

**22.** Utilisation d'une composition comprenant un glucane extrait de diatomées pour la production d'un constituant ou additif d'un aliment pour animaux, à des fins d'élevage de poissons, de mollusques et crustacés, de volailles, d'autres animaux et de mammifères.

**23.** Utilisation suivant la revendication 20, dans laquelle ladite composition comprenant du glucane est extraite de diatomées sous forme déshydratée.

**24.** Utilisation suivant la revendication 23, dans laquelle ladite diatomée est une Bacillariophyta sp.

**25.** Utilisation suivant la revendication 24, dans laquelle ladite Bacillariophyta sp est un Chaetoceros mulleri.

Figure 1.

Figure 2.

Figure 3.

Figure 4.

Figure 5.

Figure 6.

Figure 7.

Figure 8.

Figure 9.

Figure 10.

Figure 11.

Figure 12.

Figure 13.

Figure 14.

Figure 15.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 19608563 A1

- EP 0466037 A

### Non-patent literature cited in the description

- **D'SOUZA F.M.L. et al.** Flocculated microalgae concentrates as diets for larvae of the tiger prawn Penaeus monodon Fabricius. *AQUACULTURE NUTRITION,* 2002, vol. 8 (2), 113-120
- **RAA J. et al.** The use of immune-stimulants in fish and shellfish feeds. *AVANCES EN NUTRICION ACUICOLA V. MEMORIAS DEL V SIMPOSIUM INTERNACIONAL DE NUTRICION ACUICOLA,* 19 November 2000, 47-56
- **GRANUM E.** *Metabolism and function of Beta-1,3-glucan in marine diatoms,* January 2002, vol. I-VII, 1-48
- **Dalmo, R.A. ; Seljelid, R.** The immunomodulatory effect of LPS, laminaran and sulfated laminaran [β(1,3)-D-glucan] on Atlantic salmon, Salmo salar L., macrophages in vitro. *Journal of Fish Disease,* 1995, vol. 18, 175-185

- **Samuel, M. ; Lam, T. J. ; Sin, Y. M.** The effect of Laminaran [β(1,3)*D*Glucan] on the protective immunity of blue gourami, Trichogaster trichopterus against Aeromonas hydrophila. *Fish & Shellfish Immunology,* 1996, vol. 6, 443-454
- **Kim, Y. T. et al.** Structural characterization of β-D-(1→3, 1→6)-linked glucans using NMR spectroscopy. *Carbohydrate Research,* 2000, vol. 328, 331-341
- **Guillard, R.RL. ; Ryther, J.H.** Studies of marine planktonic diatoms. I. Cyclotella nana Hustedt, and Detonula confervacea (Cleve) Gran. *Canadian Journal of Microbiology,* 1962, vol. 8, 229-239
- **Skjermo, J. et al.** Immunostimulation of juvenile turbot (Scophthalmus maximus L.) using an alginate with high mannuronic acid content administered by the live food organism Artemia. *Fish & Shellfish Immunology,* 1995, vol. 5, 531-534